# EUROPEAN PATENT APPLICATION

(11) **EP 1 391 207 A1**
(43) Date of publication of application: **25.02.2004**
(21) Application number: 02724325.2
(22) Date of filing: 24.04.2002
(51) Int. Cl.: A61K 35/58, A61K 35/64, A61K 35/12

(54) **USE OF A PHOSPHOLIPASE A2 FOR THE PREPARATION OF PHARMACEUTICAL AND/OR COSMETIC COMPOSITIONS FOR THE LOCAL AND/OR SYSTEMATIC TREATMENT AND/OR PREVENTION OF DISEASES AND/OR PROCESSES CAUSED BY INTRA- AND EXTRACELLULAR PATHOGENS EXPRESSING MEMBRANE PHOSPHOLIPIDS**

(30) Priority: 24.04.2001 AR 0101891; 11.12.2001 AR 0105751
(71) Applicant: Costa, Luis Alberto, Buenos Aires (AR); Garcia Villarrubia, Vicente, 28220 Majadahonda (ES)
(72) Inventor: Costa, Luis Alberto, Buenos Aires (AR); Garcia Villarrubia, Vicente, 28220 Majadahonda (ES)
(74) Representative: Curell Aguilà, Marcelino
(86) International application number: PCT/ES2002/000198
(87) International publication number: WO 2002/085391

(57) **Abstract**

This patent relates to the use of phospholipases, e.g.: sPLA₂, as the active agent in the preparation of pharmaceutical and/or cosmetic compositions for the treatment of pathogenias mediated by micro-organisms, having a cover or membrane comprising phospholipids, particularly glycerophospholipids. In particular, it relates to the use of sPLA₂ from the venom of *Crotalus durissus terrificus* as a biocide component for the preparation of compositions for the treatment of viral infections, in particular the conditions of bacterial and parasitic acquired immunodeficiency HIV-1 and HIV-2 infections, such as parasitic conditions mediated by parasites from the plasmodium and leishmania species.

## Description

### Field of the Invention

This invention is related to the use of certain phospholipases, sPLA₂ in the formulation and preparation of pharmaceutical compositions, respectively cosmetic compositions. In particular, this invention is related to the use of sPLA₂, especially to sPLA₂-II from the venom of *Crotalus durissus terrificus*, in the formulation and preparation of compositions for the treatment of pathogenias mediated indistinctively by germs and/or modified animal and human cells, which express membrane phospholipids, such as glycerophospholipids. Said germs and cells include particularly tumoral cells, cells transformed by intracellular pathogens and, furthermore, extracellular germs such as streptococcus and pneumococcus. Other germs include viruses in general and, in particular, the human immunodeficiency viruses HIV-1 and HIV-2, bacteriae, such as microbacterium tuberculosis and leprae, and parasites of the plasmodium and leishmania species.

### Background of the Invention

In spite of their toxicity, the venom from certain ophidii, for example, from the aggressive species such as *Naja nigricolis, Naja naja atra, Crotalus durissus terrificus,* and from batrachians such as *Bothrops asper,* etc., have been considered useful not only for preparing anti-ophidic serum, but also for the treatment of certain pains (1a).

On the other hand, and as a result of research linked to the treatment of tumours, their field of application has been extended to obtain certain fractions and drugs that have unexpected specificity in carcinotherapy techniques (1a), increased specificity and efficiency in combination with other venom or fractions thereof from different sources (2a).

Said research has led to the separation and identification of a sediment factor or component from oncological activity; it is a segregated 2-phospholipase, in other words, segregated phospholipase A₂ (sPLA₂)-(2), whose anti-oncogenic activity has been confirmed and demonstrated by Luis A. Costa et al. (3a).

The field of application of sPLA₂ separated from the venom of the mentioned species and from native venom has been extended with the research conducted by David Fenard et al (4) who have ascertained that certain phospholipases A₂ separated from other vectors such as *Naja naja mossambica, Naja nigricolis,* have potent inhibitory activity against both the replication and the invasive capacity of HIV-1 and HIV-2, by blocking certain membranic receptors in human cells. Said research relating to the discovery that these lipases act according to mechanisms other than the anti-retroviral mechanisms known and applied to date, leading to the improvement of current treatments by reducing the rate of resistance.

This finding and the empirical confirmation thereof provide an important innovation in the treatment and profilaxes of viral infections mediated by HIV-1 and HIV-2. However, the results thereof are not comparable, since they do not justify envisaging and confirming that all sPLA₂ have or share the inhibitory activity discovered by Fenard et al. (22). In the case of *Naja Naja mossambica*, this limitation is illustrated on page 2, lines 1 to 10 (op.cit) reiterated on page 9, lines 1 to 4 of the same publication.

Phospholipases A₂ (PLA₂) are enzymes that catalyse the hydrolysis of fatty acids in position sn-2 of phosphoglycerides (1,2) in mammal cell and tissue membranes (3). Two sPLA₂ families are identified (4): cytosolic (cPLA₂), of PM:∼85 kDa associated with the metabolism of certain fatty acids: for example, arachidonic acid (5) and segregated (sPLA₂), of PM:∼14-28 kDa, active in the extracellular medium, associated with the pathogenia of infectious/inflammatory processes; *inter alia:* sPLA₂-IIA and sPLA₂-1B, recognised in synovial and pancreatic secretions, respectively. Specifically, sPLA₂-II catalyse the hydrolysis of glycerophospholipids, separating the fatty acid from position 2 and forming lysophosphoglycerides, hydrolysable in a subsequent step by specific lysophospholipases and by separating the remaining fatty acid.

The following diagram illustrates the enzymatic stages which take place in the biodegradation of phosphoglycerides: (5a) 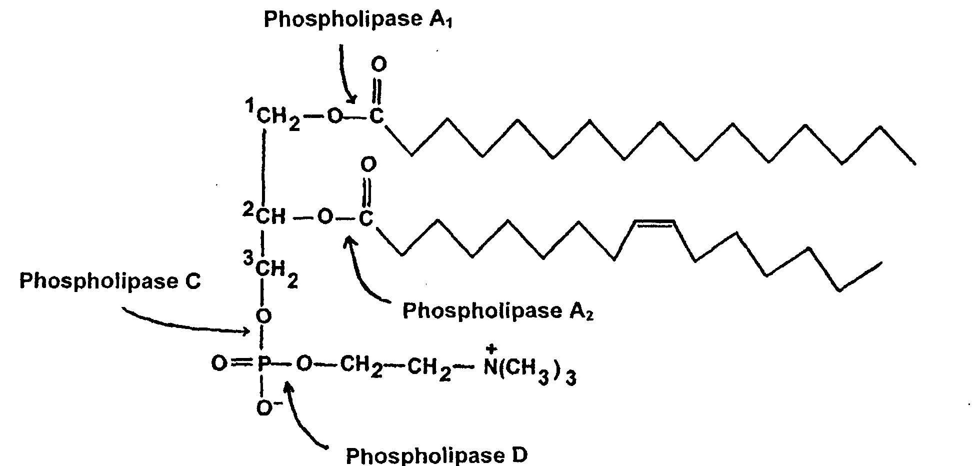
- (phospholipases A₁, A₂, C, D): A₁ and A₂: fatty acids (commonly A₂ is oleic acid or arachidonic acid)
R⁺: remainder of an aminoalcohol (serine, choline, ethanolamine)

The sPLA₂ play an important role in various processes essential to mammalian life; including the immunological responses in infectious processes, for which said enzymes have been assigned a fundamental role in certain therapies related to the treatment of infectious pathologies. In accordance with this interpretation, the sPLA₂-II would act as a link between innate and acquired immunity mechanisms, as a factor that activates the production of IFN-γ induced or mediated by IL-2 in lynphocytes (24) and, since IFN-γ induces the production of sPLA² in general (25, 26), it is considered that the existence of feedback cycles between IFN-γ and IL-2 determine possible therapeutic activity, for infectious pathologies.

Human sPLA₂-II are produced mainly in the liver, a process mediated by proinflammatory cytokines; e.g. IL-1 and IL-6 and the tumoral necrosis factor (TNF-α). Moreover, sPLA₂ interact with N(3) type receptors expressed in the brain, for example, which have great affinity with certain bee venom, and also M type receptors, expressed in the brain and lungs respectively. M receptors are notably similar to the mannose receptors expressed by the macrophagi that cause phagocytic activation, the production of cytokines in the macrophagi, which suggests that the macrophagi are activated by sPLA₂-IA, cobra venom, *Naja mossambica* and sPLA₂-IIA. Moreover, said receptors form a physiological target for sPLA₂-IB and IIA.

On the other hand, sufficient information is available on sPLA₂-II showing the importance of these enzymes in regulating the homeostasis mechanisms in various organs in the presence of certain infections, including aggressions, and consequently sPLA₂-II are attributed to participating to a certain degree in innate immunity (5) depending on the following:
1^{st}) A large part of the bactericidal activity in tears is due to their high sPLA₂-II content (6, 7). In tears, said content is 1451.3 µg/L (8) and in seminal liquid said content is in the order of 15000 µg/L (9), which values indicate anti-bacterial protection, possibly in connection with other bactericidal proteins, lactoferrin, etc.
2^{nd}) The presence of sPLA₂-II produced by secretory epitelial cells in the prostate and which indicate a possible bactericidal or viral function in seminal liquid.
3^{rd}) The important catalytic activity of sPLA₂ on bacterial phospholipids, non cytotoxic for macrophagi (4), which suggests a certain inability of sPLA₂-II, in physiological conditions, to self-attack.
4^{th}) sPLA₂ deficient mice are sensitive to infections mediated by S. aureus, but sPLA₂ transgenic mice have increased resistance to the same germ (5).

Moreover, the positive role of sPLA₂-II in certain anti-infectious resistance mechanisms, is considered possible, since patients with severe infections (peritonitis and septicemia) have significantly higher levels of sPLA₂-II than patients who have signs of inflammation without infection, which indicate a positive response to eliminating infectious agents. Furthermore, in patients who have extensive burns, the sPLa₂ levels increase only when infection appears, which excludes the intervention of factors or mediators peculiar to the patient, and would confirm the participation of factors peculiar to the infectious agent, possibly membranic bacterial lypopolysaccharides (LPS).

This hypothetical anti-microbial role of sPLA₂-II has been confirmed in different experiments: (i) the sPLA₂-II in inflammatory fluids has potent bactericidal activity against Gram positive bacteriae (18) in response to the activity of the bacterial membrane; and the proved additive effects of sPLA₂-II against β-lactamic antibiotics used in subinhibitory doses (10). Furthermore, the following has been demonstrated: i) the *in vitro* and *in vivo* (5) bactericidal potency of sPLA₂-II against S. aureus (5); and ii) that serum from E.coli infected bovine has important bactericidal activity against S. aureus, Streptococcus pyogenes and capsulated E.coli, which is attributable to sPLA₂-II (11) owing to the blockage by monoclonal antibodies aimed against human sPLA₂-II, said activity being restored by adding same.

To summarise: certain sPLA₂-II have innate anti-infectious activity not aimed against all known germs: bacteriae, viruses, fungi and parasites; e.g.: sPLA₂-II act directly against Gram-positive bacteriae; however, they alone do not have germicide activity against Gram-negative bacteriae, which is attributable to their inability to cross cellular membranes, the laminar network of peptidoglycans protecting membranic phospholipids (20); if said wall is destroyed by other plasmatic factors in the host, which are present in the medium, sPLA₂-II have germicide activity, although in reduced concentrations. One of said factors is the bactericidal protein which increases the permeability ("bactericidal/permeability-increasing protein" (BPI)) and attack complex of the membrane belonging to the complement system.

Moreover, the intervention of sPLA₂ has been proved in cases of malaria and in infections mediated by fungi, which suggests that these enzymes participate in the pathogenia or in the resistance to fungal and parasitic infections. Given that sPLA₂-II induce the endogenic production of the enzyme lysosomal β-glucuronidase, this being the enzyme responsible for the degradation of some intracellular parasites, the possible therapeutic role of sPLA₂-II in some infections caused by said parasites is understandable.

The results of recent experiments related to the *in vitro* inhibitory activity of certain sPLA₂-II, obtained from animal venom, on HIV (*vide supra* Fenard et al.), reveal that only four out of eleven assayed sPLA₂ appear to protect certain cells from HIV-1 replication (12), and these four active, animal venom sPLA₂ belong to the group sPLA₂-II. It is worth mentioning that neither the porcine (pancreatic, IB) or the human recombinants (pancreatic, IB group and 1A group) reveal any anti-viral effect (22); and said active sPLA₂-II were separated from bee venom, the venom of the cobra *Naja mossambica mossambica*, taipoxin and nigexine from the venom of the cobra *Naja nigricollis,* and also crotoxin from the venom of the *Crotalus durissus terrificus,* whose anti-HIV activity has been assessed by the applicants (13).

These studies have led to the deduction of a series of facts that are essential for understanding the biological activity of sPLA₂-II:
1^{st}) That the viral effects of sPLA₂ are selective. Not all sPLA₂ are active against HIV, this diversity being proved by the following experiment: the study conducted with monoclonal antibodies directed against human sPLA₂-II reveals that there are no crossed reactions with human pancreatic PLA₂ or with PLA₂ from the venom of *Crotalus durissus terrificus* and, moreover, mammalian sPLA₂-II do not interfere with the anti-HIV activity of those sPLA₂-II obtained from animals (12).
2^{nd}) Although the HIV membrane essentially comprises glycerophospholipids, the main substrate of sPLA₂, the anti-HIV activity thereof is not due to catalytic effects on the lipids of the virus membrane, since they respond to interactions on specific receptors in the HIV membrane.

Some phospholipids without catalytic activity, as is the case of Ba IV from the venom of *Bothrops asper,* have a weak anti-HIV effect, and adding sPLA₂ inhibitors does not have any effect on cell infection; furthermore, the sPLA₂ anti-HIV activity is not due to cytotoxic effects on infected cells (12, 13). Here, it is deduced that sPLA₂ having anti-HIV activity work with specific receptors. These receptors appear to be of the N type, which are expressed in tissues and cells, including the immunological kind (3). Moreover, other experiments show that the anti-HIV effects of certain sPLA₂ are associated with other N receptors.

3^{rd}) Some assays show that sPLA₂-II act after the binding of the virus to the cells but before the reverse transcription complex (RTC) is released.

It is worth remembering that sPLA₂-II which are active against HIV, do not interfere with the CD4/gp 120 binding, or with the formation of cellular syncyti and, in spite of this, they are potent inhibitors against the virus entering the cell.-These latter effects, assessed by the intracytosolic detection of Gag p24, are similar to those obtained with the chemokine SDF-1 that blocks this binding. Therefore, some sPLA₂ appear to respond to their ability to prevent the disassembly of the RTC from the virion in the membrane of the infected cells, thus preventing this complex from reaching the cell core. However, there is no knowledge of the events that occur once the sPLA₂-II bind to the N receptor to prevent the disassembly of the RT complex (2).

This is interpreted by the applicants in the sense that the anti-HIV effects of sPLA₂ are related to the processes for fusing the virus to the host cell membrane, since it is known that HIV-1 infection begins with the interaction of the gp120/gp41 complex of the viral membrane with at least two host cell receptors: the CD4 receptor and a member of the family of receptors for chemokines; next, the gp120/gp41 complex in response to conformational changes leading'to the fusion of the viral membrane to the host cell membrane. Then, the RTC is released, which migrates towards the cell core carrying all the necessary information to begin the synthesis of proviral DNA, following the DNA integration in the host.

The molecular bases of specific HIV-1 tropism reside in the ability of the gp120/ complex to interact with a receptor from the chemokine family; whereby the HIV-1 strains showing macrophagi tropism replicate therein and in TCDA4+ cells by means of binding to the CCR5 receptor, and are the so-called R5 virus. On the contrary, the strains showing tropism by T lynphocytes, replicate in TCD4+ lynphocytes by means of the CXCR4 chemokine receptors, and are the X4 virus. Normally, the R5 viruses do not induce the formation of cellular syncyti, while the X4 virus do induce the formation thereof.

The pathogenic and therapeutic importance of chemokine receptors is sufficiently supported in experiments since, in use, derivatives of chemokine CC or CXC, or small molecules that bind to different co-receptors, behave like HIV inhibitors.

From the detailed observation of the afore-mentioned processes of cellular fusion and the side effects of anti-retroviral therapies, possible mechanisms are deduced for which some sPLA₂ could have anti-HIV effects. The new developments in the physiopathology of HIV-1 infection have brought to light the fact that, apart from the afore-mentioned receptors, some substances, such as cholesterol, act as co-receptors for chemokine receptors, since the breakdown of the cholesterol in the host cell membrane by cyclodextrines, prevents the formation of cellular syncti, and *in vitro* infection by R4 virus and R5 virus (14). Taking into account the affinity between sPLA₂-II from the venom of *Crotalus durissus terrificus* and cholesterol (15), it is possible to envisage that at least partially the anti-HIV activity of said sPLa₂-II (13) is due to its ability to modify or block these cholesterol co-receptors.

Another important aspect of HIV infection, and the hypothetical role played by sPLA₂, is related to the function of cyclophilin A (CypA). This isomerase protein on the host and present in the HIV cover membrane favours the disassembly of the RTC therefrom, whereby the RTC reaches the cell core. Other authors have shown that the CypA favours the binding of the HIV-1 virus to the host cells owing to its ability to attach to heparans, through domains rich in basic remains similar to the motives binding heparin; next, the fusion of gp 120/CD4 takes place. Then, given the affinity between sPLA₂ and heparans, it is understood that these enzymes could interfere in the binding of the HIV virus to the host cell membrane. Therefore, certain sPLA₂-II originating from mammals or animal venom, produce germicide effects on bacteriae, fungi and parasites, directly mediated by the catalytic effects of sPLA₂-II on the cell membranes, in response to the binding thereof to specific membrane receptors. However, and in spite of the more than possible participation of sPLA₂-II in the pathogenia of some viral infections, none of the human sPLA₂-II under study reveal direct activity against the HIV viruses(12). However, certain sPLA₂-II separated from animal venom, e.g.: bee venom, the venom of *Naja mossambica mossambica, Naja nigricollis, Bothrops asper* (12) and *Crotalus durissus terrificus* (13), have potent anti-HIV activity. Out of these ones and, for example, the sPLA₂ from *Crotalus durissus terrificus,* even produces positive effects *vis á vis* tumours susceptible of viral ethyology. As we have indicated, the mechanism(s) according to which sPLA₂-II would behave as HIV replication inhibitors is due to their capacity to attach to the cholesterol and heparans in the host cell membranes, thus preventing the attachment of chemokines and CyPA in the virus cover membrane. Therefore, and this is the basis of this invention, this anti-microbial, and above all ant-iviral, activity of certain sPLA₂-II would be due to germicide effects already known (vide supra) and bearing in mind the extraordinary ubiquitousness and operational diversity of some sPLA₂-II , the applicants consider that some are closely connected to initiating and/or maintaining specific immunological responses, which would mean sPLA₂-II are enzymes capable of acting as real links between innate (natural) and adaptive or acquired (specific) immunity mechanisms.

In relation to this hypothesis, it is obvious that sPLA₂-II play an active roll in the production of interferon gamma (IFN-γ) induced by interleukin 2 (IL-2) in lynphocytes (34). In turn, IFN-γ induces the production of PLA₂, which appears to lead to the creation of a feedback circuit between both mediators, which exists as long as endotoxin remains, which would explain the correlation between the levels of both mediators during the lethal sepsis caused by Gram negative bacteriae. Given the capacity of IFN-γ to self-expand in sites far from its origin, one can understand the systemic affectation caused by the septic process, in consideration of the role of IFN-γ in anti-bacterial and anti-viral resistance, this can be explained by the fact that the increase thereof strengthens the host's anti-infectious resistance. However, the role of IFN-γ in initiating the specific responses mediated by the T "helper" 1 lynphocyte (Th1) is now evident, and clearly seems to be to associate sPLA₂ with the adaptive immunity mechanisms.

Finally, today there is no doubt regarding the important role of the natural killer cells (NK) in host resistance mechanisms against aggression. Their anti-bacterial and anti-viral activity is known, as well as their involvement in the anti-tumoral resistance mechanisms, which are essentially of the anti-metastasic type. Equally, the NK cells act as true links between the innate and adaptive immunity mechanisms.

Activation of the NK cells is triggered by several endogenic factors, *inter alia,* interleukins 1 (IL-1), 2(IL-2) and 12 (IL-12) and interferon alpha (IFN-α) and interferon gamma (IFN-γ), including cytokines. As for the possible relation between sPLA₂ and NK cells, it is known that treatment with some PLA₂ inducers causes an inflammatory reaction that is capable of inducing the regression of experimental glyomes in rats.

Furthermore, tumoral regression coincides with increases in the levels of PGE2 and leukotriene B4 (LTB4), as well as with the presence of NK cell infiltrates at the site of tumoral regression (16), which, when less, suggests the ability of sPLA₂ or metabolites produced thereby (PGE2 and/or LTB4) to mobilise NK cells to the tumour site. More recently, other authors have shown the involvement of PLA₂ and some metabolites, essentially LTB4, in the mechanisms of cellular lysis mediated by NK cells (31). In this sense, the same group describes in another study how occupation of NK cells by the CD16 receptor causes therein the activation of cPLA₂ and the secretion of sPLA₂, although neither PLA₂ appear to play an important role in the release of granules, said sPLA₂ being responsible for the lysis of the target cell (18). On the contrary, when occupied by another receptor NK (NKR-P1A) the same effects are produced on the cited PLA₂ (cytosolic and segregated) but with release of the granules that are entrusted with the lytic function of NK cells (19). Finally, other authors clarify this situation showing that the activation of the NK cells is first triggered by the presence of lysophospholipids: lysophosphatidylcoline, arachidonic acid, or products of the lypoxygenase pathway present in the target cell membranes, which subsequently leads to the activation of cPLA₂ and sPLA₂ in NK cells and the consequent release of granules (20). To summarise, sPLA₂ produced by the NK cells proper play an essential role in the activation mechanisms thereof. However, the consulted biography does not contain information showing that the exogenic addition of sPLA₂ is capable of activating NK cells, in this application, the sPLA₂ from the *Crotalus durissus terrificus,* including effector lynphocytes. The information obtained during the experiments detailed below sufficiently explains and justifies this anti-infectious activity.

The interpretation and assessment of the state-of-the-art experiments, which are considered and assessed in the preceding text, together with the experimental work carried out by the applicants, summarised in Example 1 and the following Tables and Graphs, leads to a new application of certain phospholipases A2, specifically sPLA₂ from the venom of *Crotalus durissus terrificus* as the composition agent for treating infectious conditions mediated by micro-organisms, the membrane of which includes glycerophospholipids as a structural component.

It is worth adding that, in mammals, the synthesis of phospholipids, phosphoglycerides in sphyngolipids, occurs essentially in the surface of the endoplasmatic reticule, and in part the synthesised phospholipids remain on the surface of said reticular structure, but mostly they migrate and the poles are inserted into the membrane structures in different proportions. The mechanism whereby synthesised phospholipids have a specific destination, is not known; furthermore, they move and position themselves driven by vesicles in the Golgi apparatus, a mechanism in which cytosolic proteins also intervene (Lehnning et al; op. cit).

Therefore, since the phospholipase sPLA₂ (3a) from the venom of *Crotalus durissus terrificus*, and from the complexes made up of Crotoxin A and Crotoxin B, when combined with the cardiotoxin from *Naja naja atra,* have sufficient viral activity to prevent contamination, e.g.: the proliferation of HIV-1 and HIV-2 *in vitro* in human cells, the applicants have considered the possibility, which is later justified in experimentation, of using both phospholipase sPLA₂ from crotoxin and the crotoxin complex and the afore-mentioned combinations thereof, as anti HIV-1 and anti-HIV-2 agents.

In support of this work hypothesis there is the information showing the low presence of sPLA₂-II in viral infections, which is the contrary to what happens in patients with sepsis and in patients with non septic bacterial infections (6), and leads to the opinion that the absence of sPLA₂ is related to a certain viral escape mechanism. Bearing in mind that the sPLA₂-II is essential for the production of interferon gamma (IFN-γ) induced by interleukin 2 (IL-2) in lynphocytes (7), and given the role of IFN-γ in anti-viral and anti-bacterial resistance, either directly (8), or through the induction of the so called natural killer cells (NK) (9, 10) one can understand the crucial role these cells play in anti-viral resistance mechanisms, including the HIV virus. Equally, IFN-γ induces the production of PLA₂ (11, 12), which leads to the creation of a feedback circuit, which would allow a good level of, anti-viral resistance to be maintained. Furthermore, the ability of IFN-γ to self-expand in sites remote to its origin (13) implies the existence of a systemic state of anti-viral resistance. The quantitative information documented herein corresponds to information corresponding to: 1^{st}) *in vitro* anti-HIV activity of the crotoxin complex and sPLA₂ and 2^{nd}) the strengthening activities of the crotoxin complex and sPLA₂ on the behaviour of NK cells.

### Summary of the invention

Therefore, the main object of this invention, the use of phospholipase, particularly sPLA₂-II, for preparing pharmaceutical and/or cosmetic compositions comprising effective amounts of said complex as the active agent in combination with a biocompatible carrier, for the treatment of infectious conditions mediated by pathogenic germs that express phospholipids, e.g.: glycerophospholipids, as structural components of the membrane thereof.

Another object of said invention is the use of the crotoxin complex to prepare pharmaceutical and/or cosmetic compositions comprising effective amounts of said complex as the active agent in combination with a biocompatible carrier, for the treatment of infectious conditions mediated by pathogenic germs that express phospholipids, e.g.: glycerophospholipids, as structural components of the membrane thereof.

Yet another object of this invention is the use of the crotoxin complex from *Crotalus durissus terrificus* in the preparation of the afore-mentioned pharmaceutical and/or cosmetic compositions.

Furthermore, an object of this invention is a method for the local and/or systemic treatment of infectious conditions mediated by pathogenic germs, which comprises treating patients showing signs of infection mediated by pathogenic germs that secrete glycerophospholipids with pharmaceutical and/or cosmetjc compositions that comprise effective amounts of said complex.

### BRIEF DESCRIPTION OF THE FIGURES

Figure 1 shows the results obtained through triple fluorescence in flow cytometry of the *in vitro* effects of VRCTC-310-ONCO on human natural killer cells (NK) (CD3-CD56+CD4-CD8-) separated in peripheral blood.
Figure 2 shows the results obtained by through triple fluorescence in flow cytometry of the *in vitro* effects of VRCTC-310-ONCO on human cytotoxic lynphocytes (LTC) (CD3+CD56+CD4-CD8+) separated in peripheral blood.
Figure 3 shows the results obtained through triple fluorescence in flow cytometry of the *in vitro* effects of VRCTC-310-ONCO on human T "helper" lynphocytes (Th) (CD3+CD56-CD4+CD8-) separated in peripheral blood.

### Detailed description of the invention

The use of phospholipases recognises different variants in the field of pharmacology; *inter alia* the sPLA₂ from the venom of *Crotalus durissus terrificus* has already been proposed and used in the treatment of neoplasias (27, 28). This invention provides a new application of said enzymes, in particular sPLA₂ which can be separated from said venom, based on the results obtained in the experiments carried out by the applicants in the treatment of certain infections, using said enzymes as the active agent.

Therefore, it is related to the indistinctive use of said sPLA₂ phospholipases, for formulating and preparing pharmaceutical and cosmetic compositions, which can be administered by the most appropriate way and methods and in accordance with the conditions and the state of the patients and the nature of the infectious agent. Preferably, it refers to the use of sPLA₂ from *Crotalus durissus terrificus* in the preparation of compositions which, for example, can be injected, with preventive intentions. Evidently, in all cases and situation, the doses, application programs, etc., will depend on the patient's condition, such as the virulent nature of the pathogenic agent, the acuteness of the infection, etc., these conditions being subject to the interpretation and decisions of the professional or professionals in charge of the case.

The use of the crotoxin complex and the segregated phospholipase A₂ (sPLA₂) forming part of said complex, in accordance with that described herein and claimed below, comprises the preparation of indistinctive pharmaceutical and/or cosmetic compositions in which the above intervene as the conveniently carried active agent, and coadjuvants including common excipients, diluents, etc., for the preparation of tablets, capsules, injectable substances (intramuscular, subcutaneous, etc.), patches, ointments, etc. In practise, the formulation and preparation of said compositions implies using diluting, agglomerating or disintegrating fillers, as the case may be; resorption accelerators, lubricants, etc, well known in the conventional art.

The use of liquid carriers means formulations in solution can be prepared, application sprays, for example, of the subcutaneous, intramuscular, parietal type; topical compositions, including programmed release compositions, maintained or directed towards an organ or tissue structure, in specific physiological form. In each case, the composition, if necessary, is applied in combination with other therapeutic agents.

The term "effective amount(s)" used in the scope of this invention, is related to the administerable dosis of sPLA₂ that is capable of alleviating, eliminating the symptoms of the pathological disorders associated with the permanence and/or proliferation of the pathogenic agent: HIV, respectively, and to the doses of sPLA₂ administered to reduce the pathogenia (in other words, the population of the pathogen agent) in a programmed manner until it is cancelled out.

A typical innocuous dosis, that is without adverse side effects, is obtained by assessing the anti-HIV activity, determining the minimum inhibitory concentration (MIC), the concentration corresponding to the point in which multiplication of the HIV virus is inhibited, a technique which is well known by the specialists.

The assessment can also be made *in vitro* using a series of dosification levels by means of injections, given in peritoneal or endovenous form, or where applicable, orally, to rats innoculated with HIV. Activity is measured according to the survival rate of groups treated after the death of the non treated groups.

The following text describes experiments carried out by the applicants, in an attempt to research the viral properties of the crotoxin complex separated from the venom of *Crotalus durissus terrificus*, said experiments being specifically related to the germicide activity of the crotoxin complex and the sPLA₂ in said complex, specifically in relation to micro-organisms that express membranic glycerophospholipids.

### Example 1

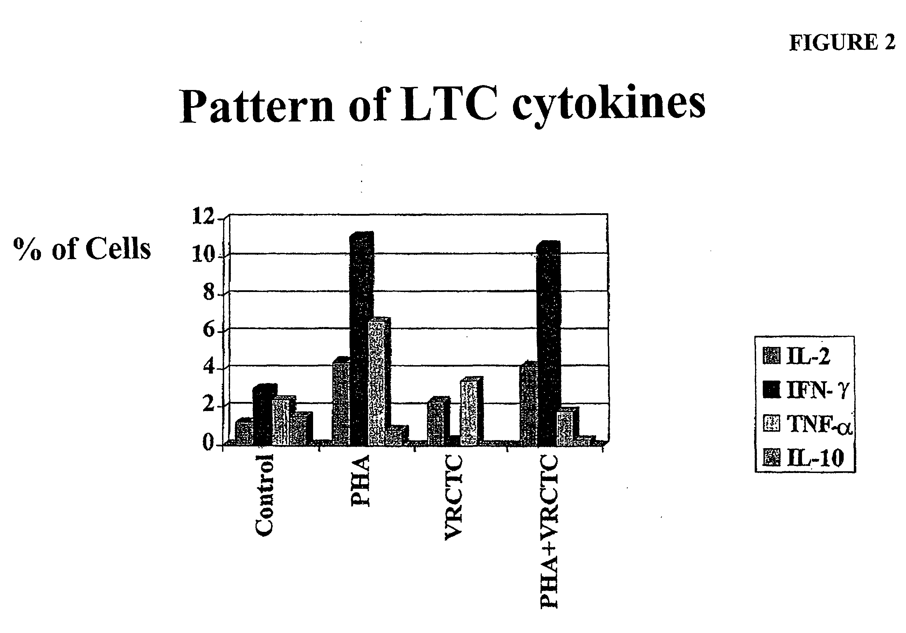

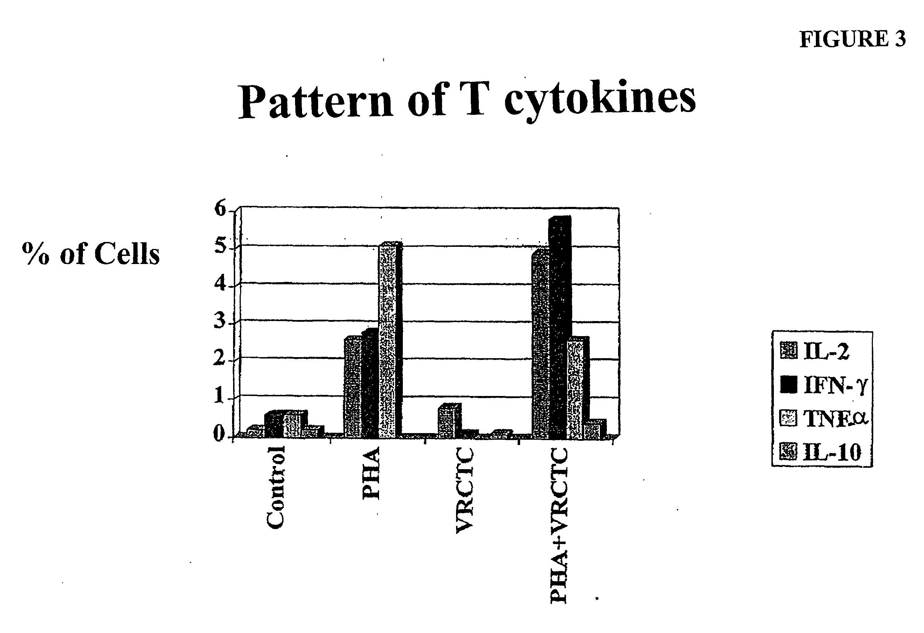

A study was conducted on the lynphocytes in peripheral blood obtained from voluntary healthy donors, aged between 18 and 30 years old. The mononucleated cells of peripheral blood were separated in Ficoll-Hypaque density gradient, according to traditional methods, and cultivated for 48 hours in the presence of 10 µg/ml of phytohemaglutinin (PHA), 1µg/ml of VRCTC-310-ONCO, or both. Following activation, the cells were dyed with monoclonal antibodies directed against the different markers, and by means of triple fluorescence in flow cytometry the expression of CD69 was observed in T lynphocytes (CD3+CD56), in cytotoxic effector T cells (CD3+CD56+) (Fig.3) and in natural killer cells (NK) (CD3-CD56+) (Fig. 2).

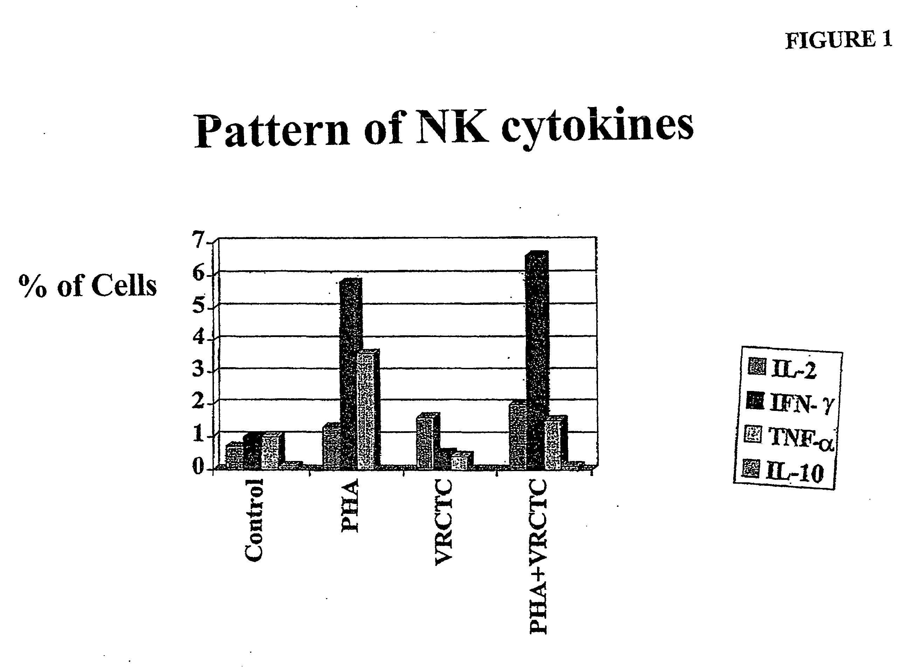

In order to detect intracellular cytokines, the cells were incubated with brefeldin for the last 6 hours of cultivation and subsequently, the expression of each cytokine was studied in relation to the expression of CD4 and CD8. To this end, monoclonal antibodies marked with FITC were used, directed against IL-2, IFN-γ, TNF-α and IL-10, which were detected by means of flow cytometry (Fig. 1).

### RESULTS

Tables 1 and 3 contain the results referring to the expression of CD69 and the various intramuscular cytokines assessed in the different populations of lynphocytes under study.

**Table 1.**

| ***In vitro* effects of VRCTC-310-ONCO on human natural killer cells (NK) (CD3-CD56+CD4-CD8-) separated from peripheral blood.** | | | | | |
|---|---|---|---|---|---|
| **% EXPRESSING CELLS** | | | | | |
| **GROUPS** | **CD69** | **IL-2** | **IFN-γ** | **TNF-α** | **IL-10** |
| **Control** | 3.1 (19.6) | 0.7 | 1 | 1 | 0.1 |
| **PHA** | 98 (221) | 1.3 | 5.8 | 3.6 | 0 |
| **VRCTC** | 46.7 (61) | 1.6 | 0.5 | 0.4 | 0 |
| **PHA+VRCTC** | 99.4 (288) | 2 | 6.6 | 1.5 | 0.1 |
| PHA: phytomaglutinin; IL-2; interleukin 2; IFN-γ; interferon gamma; TNF-α; factor alpha of tumoural necrosis/cachetin; IL-10; interleukin 10. The data in brackets indicate the values in MFC. | | | | | |

**Table 2.**

| ***In vitro* effects of VRCTG-310-ONCO on human cytotoxic lynphocytes (LTC) (CD3+CD56+CD4-CD8+) separated from peripheral blood.** | | | | | |
|---|---|---|---|---|---|
| **% EXPRESSING CELLS** | | | | | |
| **GROUPS** | **CD69** | **IL-2** | **IFN-γ** | **TNF-α** | **IL-10** |
| **Control** | 10.2 (76) | 1.2 | 3 | 2.4 | 1.5 |
| **PHA** | 95 (282) | 4.4 | 11.1 | 6.6 | 0.8 |
| **VRCTC** | 48.4 (196) | 2.3 | 0.3 | 3.4 | 0 |
| **PHA+VRCTC** | 96.6 (304) | 4.2 | 10.6 | 1.8 | 0.3 |
| PHA: phytomaglutinin; IL-2; interleukin 2; IFN-γ; interferon gamma; TNF-α; factor alpha of tumoural necrosis/cachetin; IL-10; interleukin 10. The data in brackets indicate the values in MFC. | | | | | |

**Table 3.**

| ***In vitro* effects of VRCTC-310-ONCO on human T "helper" lynphocytes (Th) (CD3+CD56+CD4+CD8-) separated from peripheral blood.** | | | | | |
|---|---|---|---|---|---|
| **% EXPRESSING CELLS** | | | | | |
| **GROUPS** | **CD69** | **IL-2** | **IFN-γ** | **TNF-α** | **IL-10** |
| **Control** | 0.5 (73) | 0.2 | 0.6 | 0.6 | 0.2 |
| **PHA** | 82 (210) | 2.6 | 2.8 | 5.1 | 0 |
| **VRCTC** | 6.3 (143) | 0.8 | 0.1 | 0 | 0.1 |
| **PHA+VRCTC** | 87.6 (252) | 4.9 | 5.8 | 2.6 | 0.4 |
| PHA: phytomaglutinin; IL-2; interleukin 2; IFN-γ; interferon gamma; TNF-α; tumor necrosis factor alpha /cachetin; IL-10; interleukin 10. The data in brackets indicate the values in MFC. | | | | | |

### Interpretation of the experiment results

From the experimental data detailed in Tables 1 and 2 and from the comparative analysis of the numeric values in Table 3, it is evident that the addition of VRCTC-310-ONCO to the cellular cultivation causes considerable increases in the expression of CD69 in NK cells (Table 1), in cytotoxic effector T cells (Table 2) and, in general, in the whole population of (T) cells (Table 3). Equally, the addition of VRCTC-310-ONCO increases slightly, or when less does not interfere with the induction of CD69 caused by PHA (Tables 1 to 3). This is the first time that it has been shown that a snake venom, in this case *Crotalus durissus terrificus*, having a sPLA₂ as its active ingredient, is able to cause *in vitro* the expression of CD69 in human lynphocytes of peripheral blood.

Receptor CD69 is one of the earliest activation antigens in human and murine lynphocytes (21). CD69 is induced *in vitro* on practically all the cells of haematological origin, although it is expressed constitutively in monocytes, platelets and Langerhans epidermic cells in humans. Today, it is considered that the expression of the activation of CD69 is a predicative sign of lynphocyte functionality' both in healthy subjects and in patients infected with HIV. In this sense, there is the generalised opinion that resting lynphocytes, including T lynphocytes CD8+ memory, do not express or express little CD69. The importance of the CD69 receptor in the host anti-infectious resistance mechanisms has been manifested in different experimental and human models. Thus, the experimental infection by *Leishmania* is incapable of inducing the expression of CD69 in treated patients. If we add to that the fact that VRCTC behaves as an inhibitor of the intracellular expression of TNF-α, or when less does not cause the expression of this cytokine (Tables 1 - 3), and that this cytokine plays a disagreeable role in promoting HIV replication and the apoptosis of NK cells, one can understand further the beneficial effects that could be derived from treatment with VRCTC in AIDS and in other infections which lead to increases in the production of this cytokine.

Finally, the behaviour of the intracellular expression of cytokines under the different assayed stimuli follows the same pattern in all the analysed cells (Tables and Figs. 1-3), thus revealing the ability of VRCTC to activate both the NK cells and the T lynphocytes, above all, those provided with cytotoxic effector functions. Bearing in mind the essential role of PLA2 in the cytotoxic activity of NK cells (17-20), the effects revealed by VRCTC on cytotoxic effector cells is not surprising, therefore, although the latter are NK or T lynphocytes.

Form the information detailed in the text above, together with the data and conclusions arising from Example 1 and the values summarised in the Tables above, it is evident that VRCTC behaves *in vitro* as an anti-microbial agent with a double action mechanism: a) a direct germicide effect on the membranes of bacteriae, viruses and parasites and b) an immunomodulating effect based essentially on its ability to induce the expression of CD69 and to mitigate the production of proinflammatory cytokines immersed in the replication of certain viruses, such as HIV, for example, coincidences and conclusions which support the novelty of the object characterised in the following claims:

### REFERENCES

**1. Waite M.** Biochemistry, molecular biology, and physiology of phospholipase
   A2 and its regulatory factors: phospholipases, enzymes that share a substrate class. Adv. Exp. Med. Biol. 1990; 279:22.
   **1a. Guillermo J. H. Plata et al.** U.S. Patent 5.164.196
**2. Mayer RJ, Marshall LA.** New insights on mammalian phospholipase A2(s):
   comparison of arachidonoyl-selective and -nonselective enzymes. FASEB J
   1993; 7: 339-48.

   **2a. Juan C. Vidal et al.** U.S. Patent 5.232.911 Official Gazette, 3 August 1993.
   **2b. Marquart et al.** U.S. Patent 4.731.439.
   **2c. Hendon:** R.A. Toxicology Biol. Abstract (Ref: 57778) Biol. Abstract 68 (9).
   **2d. W.E. Haast** U.S. Patent 4.341.762.
**3**. **Dennis E.A.** Diversity of group types, regulation, and function of phospholipase A2. J Biol Chem. 1994: 269:13057-60.
   **3 a. Luis A. Costa et al.** Anti-cancer Drugs, 1997-vol. 8; pag. 829-834.
**4. Schoenberg MH, Mayer JM, Beger HG.** Phospholipase A2-From basic
   research to clinical reality. Chirug 1997; 68: 1112-8.
**5. Lin LL, Lin AY, Knopf JL.** Cytosolic phospholipase A2 is coupled to hormonally regulated release of arachidonic acid. Proc. Natl. Acad. Sci. USA 1992; 89:6147-51.
   **5a. Tishfield JA.** A reassessment of the low molecular weight phospholipase A2 gene family in mammals. J. Biol. Chem. 1997; 272; 17247-50.
**6. Lambeau G., Lazdunski M.,** Receptors for a growing family of secreted phospholipases A2. Trends Pharmacol. Sci. 1999; 20:162-70.
**7. Cupillard L., Koumanov K, Mattei MG, Lazdunski M, Lambeau G.** Cloning, chromosomal mapping, and expression of a novel human secretory phospholipase A2. J. Biol. Chem. 1997;272:15745-52.
**8. Valentin E, Koduri RS, Scimeca JC, Carle G., gelb MH, Lazdunski M, Lambeau G.** Cloning and recombinant expression of a novel mouse-secreted phospholipase A2. J. Biol. Chem. 1999; 274:19152-60.
9. **Kaiser E.** Phospholipase A2: its usefulness in laboratory diagnostics. Crit. Rev. Clin. Lab.Sci. 1999; 36:65-163.
**10. Crowl RM., Stoller TJ, Cronroy RR, Stoner CR.** Induction of phospholipase A2 gene expression in human hepatoma cells by mediators of the acute phase response. J. Biol. Chem. 1991; 266:2647-51.
**11. Ogawa M, Arakawa H, Yamahita S, Sakamoto K, Ikei S.** Postoperative elevations of serum interleukin 6 and group II phospholipase A2: group II phospholipase A2 in serum is an acute phase reactant. Res. Commun Chem. Pathol. Pharmacol. 1992; 75:109-15.
**12. Lambeau G., Cupillard L., Lazdunski M.** Membrane receptors for venom phospholipases A2. En: RM Kini edit. Venom phospholipase A2 enzymes: structure, function and mechanism. John Wiley & Sons. Chichester, UK, 1997; pags. 389-412.
**13. Lambeau G. Garhanin J, Schweitz H, Qar J, Lazdunski M.** Identification and properties of very high affinity brain membrane-binding sites for a neurotoxi phospholipase from the taipan venom. J. Biol. Chem. 1989;264;11503-10.
14. **Lambeau G., Lazdunski M, Barhanin J.** Properties of receptors for neurotoxic phospholipase A2 in different tissues. Neurochem Res. 1991; 16:651-8.
15. **Lambeau G., Ancian P, Barhanin J., Lazdunski M.** Cloning and expression of a membrane receptor for secretory phospholipase A2. J. Biol. Chem. 1994; 269: 1575-8.
**16. Yamamoto Y, Kelin TW, Friedman H.** Involvement of mannose receptor in cytokine interleukin-1-β (IL-1β), IL-6, granulocyte-macrophage colony-stimulating factor responses, but not in chemokine macrophage inflammatory protein 1β (MIP-Iβ), MIP-2, KC responses, caused by attachment of *Candida albicans* to macrophages. Infect Immun 1997;65:1077-82.
**17. Triggiani M. M, Granata F, oriente A, De Marino V, Gentile M, Calabrese C, et al.** Secretory phopholipases A2 induce β-glucuronidase release and IL-6 production from human lung macrophages. J. Immunol 2000; 164:4908-15.
18. **Borregaard N, Cowland JB.** Granules of the neutrophilic polymorphonuclear leukocyte. Blood 1997; 89:3503-31.
**19. Kishimoto T.** The biology of interleukin-6. Blood 1989; 74:1-10.
**20. Gauldi J, Richards C, Harnish D, Landsdorp P, Baumann H.** Interferon β2/B-cell stimulatory factor type 2 shares identity with monocyte-derived hepatocyte-stimulating factor and regulates the major acute phase protein response in liver cells. Proc. Natl Acad Sci. USA 1987; 84:7251-5.
**21. Ohara O, Ishizaki J, Arita H.** Structure and function of phospholipase A2 receptor. Prog. Lipid Res 1995; 34:117-38.
**22. Fenard D, Lambeau G, Valentin E., Lefebvre JC, Lazdunski M, Doglio A.** Secreted phospholipases A2, a new class of HIV inhibitors that block virus entry into host cells. J. Clin Invest 1999; 104:611-8.
**23. Costa LA, García Villarrubia V.** Uso del complejo de crotoxina y de la fosfolipasa A2 para la preparación de composiciones farmacéuticas para la prevención y/o tratamiento de infecciones mediadas por el virus HIV-1 o HIV-2. Patente de invención. Solicitud argentina n° P01 01 01891 (Abril 24, 2001).
**24. Dalbleish AG, Beverley PC, Clapham PR, Crawford DH, Greaves MF, Weiss RA.** The CD4(T4) antigen is an essential component of the receptor for the AIDS retrovirus. Nature 1984; 312:763-7.
**25. Deng H., Liu R, Ellmeier W, Choe S, Unutmaz D, Burkhart M, et al.** Identification of a major co-receptor for primary isolates of HIV-1. Nature 1996; 381:661-6.
**26. Dragic T, Litwin V, Allanay GP, Matin SR, Huang Y, Nagashima KA, et al.** HIV-1 entry into CD4+ cells is mediated by the chemokine receptor CC-CKR5. Nature 1996; 381:667-73.
**27. Costa LA, Miles H, Araujo C, Villarrubia VG.** A pilot comparative study with VRCTC-310 for the treatment of heavily pretreated advanced cancer patients. En: Int Proc. Div., Monduzzi edit. 17^{th} Int. Cancer Congress. Rio de Janeiro (Brazil), August 24-28; 1998: 1491-4.
**28. J. C. Costa et al. EPA 0246 861.** Pharmaceutical Composition based on crotoxin, process for preparing the active component and pharmaceutical composition.
**29.** Ponzoni, M. Cornaglia-Ferraris. Interferon-γ-Stimulated and GTP-binding-proteins-mediated phospholipase A₂ Activation human neuroblasts. Biochem. I. 1993, 294:893-8.
**30.** WO:T, Levine S.I., Lawrence M. G.; Logun C.; Angus CW; Shelhamer IH. Interferon-γ-induces the synthesis and activated of cytosolic phospholipase A₂. I Clin. Invest (1994); 93:571-7.

The content and the teachings in the afore-mentioned bibliographical references are included expressly in this application for reference.

## Claims

1. Use of a phospholipase A₂, **characterised in that** it is for the preparation of pharmaceutical and/or cosmetic compositions comprising effective amounts of said phospholipase A₂ as the active agent in combination with a biocompatible carrier, for the prevention and treatment of infectious conditions mediated by pathogenic germs that express membranic glycerophospholipids.

2. The use according to claim 1, **characterised in that** said phospholipase A₂ is segregated from a crotoxin complex.

3. The use according to claim 2, **characterised in that** said crotoxin complex is obtained from the venom of *Crotalus durissus terrificus.*

4. The use according to claim 1, **characterised in that** said phospholipase A₂ is the phospholipase A₂ from bee venom.

5. The use according to claim 1, **characterised in that** said phospholipase A₂ is the human secretory phospholipase A₂.

6. The use according to any of the claims 1 to 5, **characterised in that** said compositions are ones that can be applied in the form of subcutaneous or intramuscular injections for anti-septic treatments.

7. The use according to any of claims 1 to 5, **characterised in that** said compositions are for the treatment of cutaneous infections, such as ointments, lotions and aerosols.

8. The use according to any of claims 1 to 5, **characterised in that** said compositions are ophthalmic compositions, such as a collyrium.

9. The use according to any of the claims 1 to 7, **characterised in that** said compositions are for the treatment of infections mediated by intracellular pathogen agents, such as leishmaniasia.

10. The use according to claim 9, **characterised in that** the leishmania comprises kala-azar, Allepo boil and infantile kala-azar.

11. The use according to any of claims 1 to 5, **characterised in that** said compositions are for preventing and/or treating infections produced by HIV-1 or HIV-2.

12. The use according to any of claims 1 to 8, **characterised in that** said compositions are veterinary compositions.

13. The use according to any of the claims 1 to 12, **characterised in that** said compositions comprise furthermore cardiotoxin from the venom of *Naja naja atra*.

14. Method for the local and/or systemic treatment of infectious conditions mediated by pathogen germs that express membranic glycerophospholipids, **characterised in that** it comprises treating patients showing signs of infection mediated by said pathogen germs with pharmaceutical and/or cosmetic compositions comprising effective amounts of a phospholipase A₂ according to claim 1.

15. The method according to claim 15, **characterised in that** said phospholipase A₂ is segregated from a crotoxin complex.

16. The method according to claims 15, **characterised in that** said crotoxin complex is obtained from the venom of *Crotalus durissus terrificus.*

17. The method according to claim 14, **characterised in that** said phospholipase A₂ is the phospholipase A₂ from bee venom.

18. The method according to claim 14, **characterised in that** said phospholipase A₂ is human secretory phospholipase A₂.

19. The method according to any of the claims 14 to 18, **characterised in that** said compositions are compositions that can be applied as subcutaneous or intramuscular injections for anti-septic treatments.

20. The method according to any of the claims 14 to 18, **characterised in that** it comprises the preparation of compositions for the treatment of cutaneous infections, such as ointments, lotions and aerosols.

21. The method according to any of the claims 14 to 18, **characterised in that** it comprises the preparation of ophthalmic compositions, such as a collyrium.

22. The method according to any of the claims 14 to 20, **characterised in that** it comprises the preparation of compositions for the treatment of infections mediated by intracellular pathogen agents, such as leishmania.

23. The method according to claim 22, **characterised in that** the leishmania comprises kala-azar, Allepo boil and infantile kala-azar.

24. The method according to any of the claims 14 to 18, **characterised in that** said compositions are for preventing and/or treating infections produced by HIV-1 or HIV-2.

25. The method according to any of the claims 14 to 20, **characterised in that** said compositions are veterinary compositions.

26. The use according to any of the claims 14 to 25, **characterised in that** said compositions comprise furthermore cardiotoxin from the venom of *Naja naja atra*.

27. A pharmaceutical and/or cosmetic composition, **characterised in that** it comprises a phospholipase A₂ according to claim 1, in combination with a biocompatible carrier, for the prevention and the treatment of infectious conditions mediated by pathogen germs which express membranic glycerophospholipids.

28. A pharmaceutical and/or cosmetic composition according to claim 27, **characterised in that** said phospholipase A₂ is segregated from a crotoxin complex.

29. A pharmaceutical and/or cosmetic composition according to claim 28, **characterised in that** said crotoxin complex is obtained from the venom of *Crotalus durissus terrificus*.

30. A pharmaceutical and/or cosmetic composition according to claim 27, **characterised in that** said phospholipase A₂ is the phospholipase A₂ from bee venom.

31. A pharmaceutical and/or cosmetic composition according to claim 27, **characterised in that** said phospholipase A₂ is the human secretory phospholipase A₂.

32. A pharmaceutical and/or cosmetic composition according to any of the claims 27 to 31, **characterised in that** said composition is one that can be applied in the form of subcutaneous or intramuscular injection for anti-septic treatments.

33. A pharmaceutical and/or cosmetic composition according to any of claims 27 to 31, **characterised in that** said composition is for the treatment of cutaneous infections, such as ointments, lotions and aerosols.

34. A pharmaceutical and/or cosmetic composition according to any of the claims 27 to 31, **characterised in that** said composition is an ophthalmic composition, such as a collyrium.

35. A pharmaceutical and/or cosmetic composition according to any of the claims 27 to 33, **characterised in that** said composition is for the treatment of infections mediated by intracellular pathogen agents, such as leishmania.

36. A pharmaceutical and/or cosmetic composition according to claim 35, **characterised in that** the leishmania comprises kala-azar, Allepo boil and infantile kala-azar.

37. A pharmaceutical and/or cosmetic composition according to any of claims 27 to 31, **characterised in that** said composition is for preventing and/or treating infections produced by HIV-1 or HIV-2.

38. A pharmaceutical and/or cosmetic composition according to any of claims 27 to 34, **characterised in that** said composition is a veterinary composition.

39. A pharmaceutical and/or cosmetic composition according to any of the claims 27 to 38, **characterised in that** said composition further comprises cardiotoxin from the venom of *Naja naja atra*.
